# EUROPÄISCHE PATENTSCHRIFT

(11) **EP 3 547 330 B1**
(45) Veröffentlichungstag und Bekanntmachung des Hinweises auf die Patenterteilung: **10.11.2021**
(21) Anmeldenummer: 18164521.9
(22) Anmeldetag: 28.03.2018
(51) Int. Cl.: G21F 3/02, A61B 6/10

(54) **STRAHLENSCHUTZBEKLEIDUNGSANORDNUNG**
RADIATION PROTECTIVE CLOTHING
SYSTEME DE VÊTEMENT DE PROTECTION CONTRE LES RAYONNEMENTS

(43) Veröffentlichungstag der Anmeldung: 02.10.2019
(73) Patentinhaber: Gerges, Anton, 63075 Offenbach/Main (DE); Gerges, Steve, 63075 Offenbach am Main (DE)
(72) Erfinder: Gerges, Anton, 63075 Offenbach/Main (DE); Gerges, Steve, 63075 Offenbach am Main (DE)
(74) Vertreter: Keil & Schaafhausen Patentanwälte PartGmbB

(56) Entgegenhaltungen:
- CN-A- 103 943 163
- CN-U- 204 654 972
- CN-U- 205 645 288
- KR-B1- 101 880 620
- US-A- 5 015 864
- US-A- 5 220 175
- US-A1- 2012 228 439

## Beschreibung

Die vorliegende Erfindung betrifft eine Strahlenschutzbekleidungsanordnung mit einem flexiblen Mantel, der Strahlenschutzmaterial aufweist und im Gebrauchszustand einen Innenraum mit einem unteren Rand umgibt, und mit einer Gewichtsentlastungseinrichtung.

Bei unter Strahlenbelastung durchzuführenden diagnostischen, therapeutischen oder chirurgischen Maßnahmen sind Ärzte und weiteres medizinisches Personal auf das Tragen spezieller Strahlenschutzbekleidung angewiesen, beispielsweise in Form von Röntgenschutzbekleidung. Die Röntgenschutzbekleidung verhindert, dass Röntgenstrahlen den menschlichen Körper des medizinischen Personals in unerwünschter Weise negativ beeinträchtigen. Auch in der Nuklearmedizin ist der Schutz vor radioaaktiver Strahlung erforderlich. Um eine ausreichende Schutzwirkung zu entfalten, muss das Strahlenschutzmaterial mit einer gewissen Dicke vorgesehen sein, was zu einer entsprechend großen Masse des Mantels führt. Eine Masse von 5 bis 10 kg oder mehr ist hierbei die Regel. Wenn eine Person einen derartigen Mantel trägt, bedeutet dies eine erhebliche körperliche Belastung. Diese hohe Gewichtsbelastung kann zu Erschöpfungserscheinungen, Rückenschmerzen, Körperfehlhaltungen und muskulären Verspannungen führen. Die Konzentrationsfähigkeit während Prozeduren am Patienten kann leiden. Eine fortdauernde Überbeanspruchung des Körpers kann langfristig zu Schäden führen, beispielsweise an Wirbelsäulengelenken, Wirbelkörpern und Bandscheiben.

Man hat daher bereits Maßnahmen vorgeschlagen, um einen ausreichenden Schutz vor Strahlen mit einem höheren Tragekomfort und einer geringeren Belastung zu kombinieren. So zeigt beispielsweise EP 1 052 651 B1 die Möglichkeit, das Gewicht des Mantels nicht nur von der Schulter der zu schützenden Person tragen zu lassen, sondern weitere Stützstellen vorzusehen, beispielsweise an der Hüfte im Rückenbereich der Person. Eine ähnliche Ausgestaltung ist auch aus US 3 052 799 A oder aus US 5 834 789 A bekannt. Hier soll das komplette Gewicht des Mantels von der Hüfte der zu schützenden Person aufgenommen werden. Eine ähnliche Ausgestaltung ist auch aus US 4 766 608 A bekannt.

Bei einer derartigen Ausbildung der Strahlenschutzbekleidungsanordnung trägt allerdings die zu schützende Person nach wie vor das gesamte Gewicht des Mantels, so dass die Gewichtsbelastung lediglich vom Rücken auf andere Körperteile übertragen wird, beispielsweise auf die Knie.

Eine andere Möglichkeit des Schutzes vor Röntgenstrahlen ist in US 5 220 175 A, US 5 015 864 A oder US 8 925 553 B2 beschrieben. Hier werden Röntgenschutzschilde gezeigt, hinter denen sich die zu schützende Person aufhalten kann. Allerdings ist ein derartiges Röntgenschutzschild ein Hindernis, um das die zu schützende Person herum greifen muss, wenn sie Handlungen an einem Patienten vornehmen möchte. Darüber hinaus benötigen derartige Schutzschilde relativ viel Platz, also eine relativ große Aufstandsfläche auf dem Fußboden, um ein Kippen zu verhindern, so dass es Schwierigkeiten bereitet, wenn mehrere zu schützende Personen gemeinsam Handhabungen an einem Patienten vornehmen müssen. Eine derartige Situation tritt aber vielfach bei operativen Eingriffen auf, wo man eine Fuß-an-Fuß-Situation benötigt, um gemeinsam arbeiten zu können.

Eine weitere Möglichkeit des Schutzes besteht darin, an einer Raumdecke eine Schienenanordnung oder eine Krananordnung vorzusehen, die den Mantel trägt. Eine derartige Möglichkeit ist in US 8 558 204 B2 oder US 2009/0256044 A1 gezeigt. Die Schienenanordnung oder Krananordnung kann zwar das Gewicht des Mantels vollständig aufnehmen. Sie schränkt aber die Bewegungsmöglichkeiten der zu schützenden Person ein, weil diese sich nur innerhalb der Reichweite der Schienenanordnung oder Krananordnung bewegen kann. Insbesondere dann, wenn ein Raumwechsel erforderlich ist, ist eine derartige Röntgenschutzanordnung nur mit einem erhöhten Aufwand zu gebrauchen.

Eine Strahlenschutzbekleidungsanordnung der eingangs genannten Art ist beispielsweise aus US 8 674 330 B2 bekannt. Hier ist der Mantel an einem Gestell aufgehängt, das sich mit vier Rollen auf dem Fußboden abstützt. Die vier Rollen müssen eine relativ große Aufstandsfläche bilden, um ein Kippen zu verhindern.

Eine ähnliche Ausgestaltung ist auch aus US 8 777 168 B2 bekannt. Hier ist der Mantel in seinem Schulterbereich an einem Ständer aufgehängt.

In beiden Fällen ergibt sich ein relativ großer Flächen- und Raumbedarf, so dass eine derartige Strahlenschutzbekleidungsanordnung in einem Operationssaal nicht genutzt werden kann, wo mehrere zu schützende Personen relativ eng benachbart zueinander stehen müssen, beispielsweise in der oben genannten Fuß-an-Fuß-Situation. Darüber hinaus schränkt eine derartige Strahlenschutzbekleidungsanordnung in vielen Fällen die Bewegungsmöglichkeiten der zu schützenden Person ein.

Der Erfindung liegt die Aufgabe zugrunde, einen möglichst hohen Strahlenschutz mit einer geringen mechanischen Belastung der zu schützenden Person zu kombinieren.

Diese Aufgabe wird mit einer Strahlenschutzbekleidungsanordnung mit den Merkmalen des Anspruchs 1 gelöst.

Bei einer derartigen Strahlenschutzbekleidungsanordnung kann das Gewicht des Mantels über die Stützeinrichtung praktisch vollständig in den Fußboden oder eine andere Unterlage, auf der die zu schützende Person steht, beispielsweise ein Podest an einem Operationstisch, abgeleitet werden. Die Stützeinrichtung steht damit nicht nach außen über den Mantel vor und behindert damit nicht das enge Zusammenstehen von mehreren zu schützenden Personen. Auch ist das Risiko gering, dass die Stützeinrichtung bei einer Bewegung der zu schützenden Person gegen einen anderen Gegenstand, beispielsweise einen Operationstisch oder dergleichen, anstößt. Der Mantel ist auch nicht mehr nur im Schulterbereich an dem Stützskelett aufgehängt, sondern hat mehrere Befestigungspunkte, die in Schwerkraftrichtung verteilt sind. Diese vermindern das Kipprisiko der Strahlenschutzbekleidungsanordnung. Die zu schützende Person kann also praktisch vollständig vom Gewicht des Mantels entlastet werden und muss lediglich geringe Kräfte aufbringen, um einen in Schwerkraftrichtung im Wesentlichen vertikal stehenden Mantel gegen Kippkräfte abzustützen, die, wie oben ausgeführt, aufgrund der verteilten Befestigung des Mantels am Stützskelett gering sind.

Die Erfindung wird im Folgenden anhand einer Röntgenschutzbekleidungsanordnung beschrieben. Röntgenstrahlen sind hierbei eine besondere Unterart von Strahlen allgemein. Dementsprechend ist die Erfindung auch allgemein bei anderen Strahlen anwendbar, die für einen Menschen gefährlich sein können.

Vorzugsweise ist das Stützskelett beweglich ausgebildet. Da das Stützskelett nicht starr, sondern beweglich und damit flexibel ist, kann es gemeinsam mit dem Mantel Bewegungen der zu schützenden Person folgen. Man kann den Mantel und das Stützskelett auch relativ eng um den Körper der zu schützenden Person herum anordnen, so dass der Raumbedarf für die Strahlenschutzbekleidungsanordnung gering bleibt und mehrere zu schützende Personen in einer engen räumlichen Nachbarschaft zusammen arbeiten können. Auch das Stützskelett kann sich vorzugsweise an den Körper der zu schützenden Person anpassen, d.h. der vom Stützskelett umgebene Innenraum ist vorzugsweise veränderbar. Das Stützskelett kann bei einer dickeren Person einen größeren Innenraum umschließen und bei einer dünneren Person einen kleineren Innenraum. Wenn das Stützskelett beweglich, also verformbar, ausgebildet ist, ist es nicht unbedingt erforderlich, dass die Stützeinrichtung innerhalb einer Verlängerung des Innenraums in Schwerkraftrichtung angeordnet ist. Mit der Beweglichkeit des Stützskeletts wird der Bewegungsradius einer Person, die durch die Strahlenschutzbekleidungsanordnung geschützt ist, etwas erweitert, so dass man in gewissem Umfang auch zulassen kann, dass sich die Stützeinrichtung etwas außerhalb der Verlängerung des Innenraums in Schwerkraftrichtung befindet.

Vorzugsweise weist die Stützeinrichtung mindestens eine Rolle auf. Die Stützeinrichtung kann damit reibungsarm über den Fußboden oder eine andere Unterlage bewegt werden, damit die zu schützende Person keine größeren Widerstände überwinden muss, wenn sie sich gemeinsam mit der Strahlenschutzbekleidungsanordnung über den Fußboden bewegt. Anstelle einer einzelnen Rolle kann man auch zwei, drei, vier oder mehr Rollen verwenden. Diese sind dann vorzugsweise alle innerhalb der Verlängerung des Innenraums in Schwerkraftrichtung angeordnet.

In einer alternativen Ausgestaltung ist vorgesehen, dass die Stützeinrichtung eine Fußbefestigung und/oder Beinbefestigung aufweist. Die zu schützende Person kann ihre Füße oder auch ihre Unterschenkel mit der Fußbefestigung verbinden. Wenn die zu schützende Person dann ihre Füße bewegt, wird die Stützeinrichtung mitgenommen. Im Übrigen leitet die Stützeinrichtung die Gewichtskräfte des Mantels aber in den Fußboden oder eine andere Unterlage ab.

In einer bevorzugten Ausgestaltung ist vorgesehen, dass der Mantel und/oder das Stützskelett eine ausgeglichene Massenverteilung um die Stützeinrichtung aufweist. Damit befindet sich die Strahlenschutzbekleidungsanordnung bei einer vertikalen Ausrichtung in einem Gleichgewichtszustand, d.h. es gibt praktisch keine oder nur sehr geringe Kippmomente, die ausschließlich auf eine ungleiche Massenverteilung zurückzuführen sind. Leichte Kippmomente können sich dadurch ergeben, dass die zu schützende Person ihre Körperhaltung ändert, beispielsweise den Oberkörper nach vorne neigt. Die damit verbundenen Belastungen auf den Körper der zu schützenden Person sind jedoch relativ klein und können ohne weiteres aufgenommen werden. Sie sind auf jeden Fall deutlich geringer als die Belastung durch das Gewicht des Mantels.

Vorzugsweise ist die Stützeinrichtung außerhalb von Beinschwingräumen angeordnet, die sich aus dem Innenraum über den unteren Rand hinaus fortsetzen. Die Beinschwingräume sind die Räume, in denen die zu schützende Person üblicherweise ihre Beine bewegt. Die Beinschwingräume sind dabei etwas außermittig links und rechts einer Mittelebene durch die Strahlenschutzbekleidungsanordnung angeordnet. Wenn die Stützeinrichtung außerhalb der Beinschwingräume angeordnet ist, dann behindert sie die Bewegung der Beine der zu schützenden Person nicht. Beispielsweise kann die Stützeinrichtung in der oben genannten Mittelebene angeordnet sein, wenn beispielsweise nur eine Rolle vorgesehen ist. Die Stützeinrichtung kann auch mit einem längeren vertikalen Abschnitt im Rückenbereich des Mantels mit dem Stützskelett verbunden sein und lediglich in einem in Schwerkraftrichtung unteren Bereich S-förmig abgewinkelt sein, so dass auch eine Person mit X-Beinen durch die Stützeinrichtung nicht behindert wird.

Vorzugsweise ist die Stützeinrichtung höhenverstellbar. Damit lässt sich die Strahlenschutzbekleidungsanordnung an unterschiedliche Körpergrößen von zu schützenden Personen anpassen. Man kann die Höhenverstellbarkeit der Stützeinrichtung auch noch für einen anderen Zweck ausnutzen. Die zu schützende Person legt sich die Strahlenschutzbekleidungsanordnung an, ohne dass die Stützeinrichtung auf der Unterlage aufsteht. Für einen relativ kurzen Zeitraum lastet dann das volle Gewicht der Strahlenschutzbekleidungsanordnung auf den Schultern der zu schützenden Person. Die zu schützende Person kann sich dann auf die Zehenspitzen stellen oder auf andere Weise die Schultern in einem ausreichenden Maße anheben. Die Stützeinrichtung kann dann in ihrer Höhe so verstellt werden, dass sie auf dem Fußboden aufsteht. Wenn die zu schützende Person dann wieder in eine normale Haltung zurückkehrt, bei der die Füße auf dem Fußboden aufstehen, ergibt sich ein kleiner Abstand zwischen einem Schulterbereich des Mantels und den Schultern der zu schützenden Person, so dass die zu schützende Person das Gewicht der Strahlenschutzbekleidungsanordnung nicht mehr tragen muss.

Vorzugsweise ist das Stützskelett zumindest teilweise mit einem textilen Material überzogen. Dies hat mehrere Vorteile. Zum einen kann man eine ansprechende äußere Gestaltung erzielen. Zum anderen kann die Haptik verbessert werden. Insbesondere dann, wenn das Stützskelett innen im Mantel angeordnet ist, vermeidet man einen direkten Kontakt des Stützskeletts mit der Person, wenn man eine Schicht des textilen Materials zwischen dem Körper der zu schützenden Person und dem Stützskelett vorsieht.

Vorzugsweise ist das Stützskelett in den Mantel integriert. Das Stützskelett ist also von außen nicht wahrnehmbar. Es hat darüber hinaus eine stabilisierende Funktion für den Mantel bei der Aufbewahrung. Die Gefahr, dass der Mantel knickt und dadurch das Strahlenschutzmaterial beschädigt wird, wird vermindert.

Vorzugsweise sind zumindest einige Elemente des Stützskeletts längenveränderbar und/oder verformbar ausgebildet. Die verformbare Ausbildung von Elementen des Stützskeletts kann für die bewegliche Verbindung von Elementen ausgenutzt werden. Sie kann aber auch zusätzlich vorgesehen sein. Eine längenveränderbare Ausbildung von Elementen des Stützskeletts ist beispielsweise im Rückenbereich von Vorteil, um der zu schützenden Person ein Beugen des Oberkörpers zu ermöglichen.

Hierbei ist bevorzugt, dass der Mantel im Bereich zumindest eines der längenveränderbaren und/oder verformbaren Elemente eine Überlänge aufweist. Solange also das entsprechende Element seine kürzeste Ausdehnung hat, kann der Mantel hier eine "Falte" werfen. Wenn sich die zu schützende Person dann nach vorne neigt und den Rücken entsprechend wölbt, dann kann der Mantel gestreckt werden, so dass die Strahlenschutzfunktion des Mantels erhalten bleibt und keine Lücken entstehen.

Vorzugsweise ist das Stützskelett aus Metall und/oder Kunststoff gebildet. In beiden Fällen lässt sich bei Wahl entsprechender Werkstoffe ein massearmes Stützskelett bilden, das dennoch eine relativ hohe mechanische Stabilität aufweist.

Vorzugsweise weist das Stützskelett eine Fachwerkstruktur und/oder eine Lochblechanordnung auf. Beides sind Möglichkeiten, um die Masse des Stützskeletts kleinzuhalten, ohne auf eine ausreichende Stabilität zu verzichten.

Vorzugsweise weist das Stützskelett eine in Schwerkraftrichtung nach unten zunehmende lokale Belastbarkeit auf. Damit trägt man der Tatsache Rechnung, dass ein in Schwerkraftrichtung unterer Bereich des Stützskeletts insgesamt mehr Gewicht aufnehmen muss als ein in Schwerkraftrichtung oberer Bereich. Auf diese Weise lässt sich die Konstruktion des Stützskeletts nach oben hin "verschlanken". Dies ist günstig, weil die zu schützende Person üblicherweise mit ihren Händen und Armen agiert, die ebenfalls im in Schwerkraftrichtung oberen Bereich der Strahlenschutzbekleidungsanordnung angeordnet sind und dann durch das Stützskelett wenig oder sogar gar nicht behindert werden.

In einer vorteilhaften Ausgestaltung ist vorgesehen, dass zumindest eine bewegliche Verbindung zwischen Elementen des Stützskeletts Rückstellmittel aufweist. Die Rückstellmittel können beispielsweise durch eine Feder oder einen Gasdruckzylinder gebildet sein. Wenn die Rückstellmittel beispielsweise im Rückenbereich des Mantels oder des Stützskeletts vorgesehen sind, erleichtern sie das Aufrichten der zu schützenden Person aus einer gebeugten Stellung.

In einem weiteren Aspekt betrifft die Erfindung eine Strahlenschutzbekleidungsanordnung mit einem flexiblen Mantel, der Strahlenschutzmaterial aufweist und im Gebrauchszustand einen Innenraum mit einem unteren Rand umgibt, und mit einer Gewichtsentlastungseinrichtung, bei der die Gewichtsentlastungseinrichtung als selbsttragendes flexibles Stützskelett ausgebildet ist, das mit dem Mantel verbunden ist und das Gewicht des Mantels in Schwerkraftrichtung verteilt an mehreren Stellen aufnimmt, wobei das Stützskelett mit einer im Gebrauchszustand auf einer Unterlage aufstellbaren Stützeinrichtung verbunden ist.

Die Erfindung wird nachfolgend anhand von bevorzugten Ausführungsbeispielen in Verbindung mit der Zeichnung beschrieben, darin zeigen:
- Fig. 1: eine erste Ausgestaltung einer Strahlenschutzbekleidungsanordnung an einer zu schützenden Person,
- Fig. 2: die Strahlenschutzbekleidungsanordnung nach Fig. 1 mit transparent dargestelltem Mantel,
- Fig. 3: die Strahlenschutzbekleidungsanordnung nach Fig. 2 an einer Person in einer gebeugten Haltung,
- Fig. 4: eine zweite Ausführungsform der Strahlenschutzbekleidungsanordnung,
- Fig. 5: eine dritte Ausgestaltung der Strahlenschutzbekleidungsanordnung,
- Fig. 6: eine vierte Ausgestaltung der Strahlenschutzbekleidungsanordnung,
- Fig. 7: eine fünfte Ausgestaltung der Strahlenschutzbekleidungsanordnung,
- Fig. 8: eine sechste Ausgestaltung der Strahlenschutzbekleidungsanordnung,
- Fig. 9: die Strahlenschutzbekleidungsanordnung nach Fig. 8 an einer sitzenden Person,
- Fig. 10: eine vereinfachte Ausführungsform der Strahlenschutzbekleidungsanordnung,
- Fig. 11: eine schematische Darstellung zur Erläuterung einer Fuß-anFuß-Konstellation am Operationstisch,
- Fig. 12: eine schematische Darstellung eines Teils eines Stützskeletts in Fachwerkausführung und
- Fig. 13: eine schematische Darstellung eines Teils des Stützskeletts in Lochblechausführung.

In allen Fig. sind gleiche und einander entsprechende Elemente mit den gleichen Bezugszeichen versehen.

In allen Fig. zeigt die Teilfigur "A" eine Vorderansicht, die Teilfigur "B" eine Seitenansicht, die Teilfigur "C" eine Rückenansicht und die Teilfigur "D" eine Draufsicht von oben.

Die Erfindung wird im Folgenden anhand einer Röntgenschutzbekleidungsanordnung beschrieben. Sie ist aber in gleicher Weise bei anderen Strahlen anwendbar, beispielsweise im Bereich der Nuklearmedizin. Die Art des Schutzes hängt von den verwendeten Materialien ab. Man kann deswegen ohne weiteres den Begriff "Röntgenschutzbekleidungsanordnung" durch Strahlenschutzbekleidungsanordnung" ersetzen.

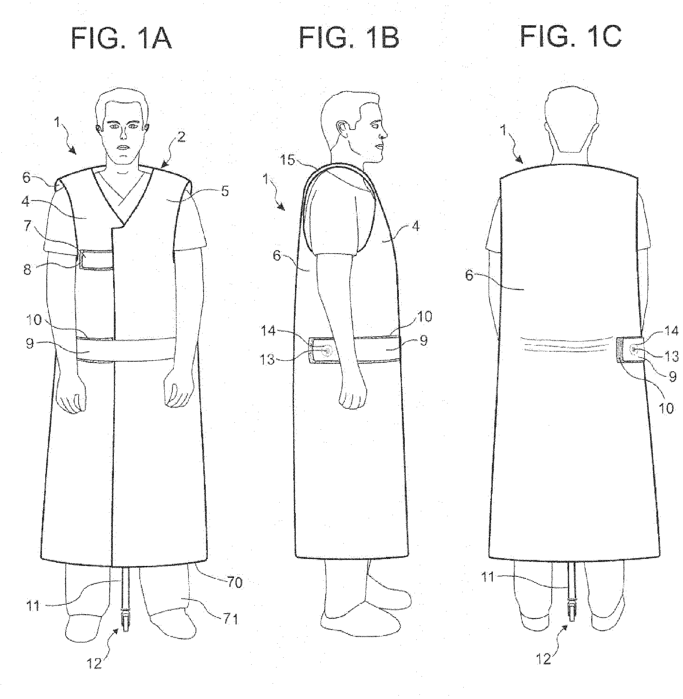

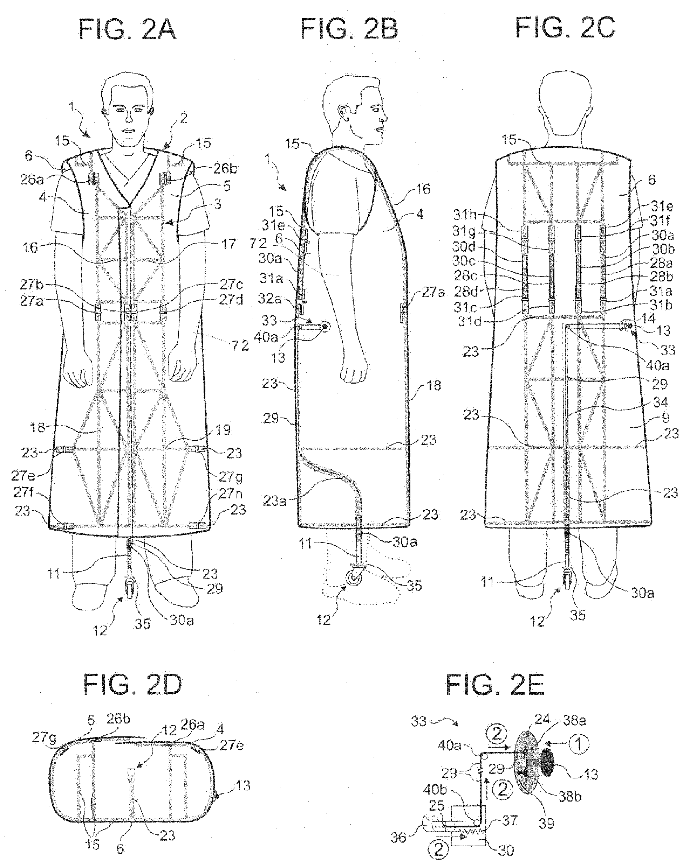

Die Fig. 1 und 2 zeigen schematisch eine Strahlenschutzbekleidungsanordnung in Form einer Röntgenschutzbekleidungsanordnung 1 mit einem flexiblen Mantel 2, der Strahlenschutzmaterial aufweist, und einem in Fig. 1 nicht, sondern nur in Fig. 2 sichtbaren Stützskelett 3. Im vorliegenden Fall ist der Mantel 2 außen am Stützskelett 3 angeordnet, so dass der Mantel 2 das Stützskelett 3 bedeckt. Das Stützskelett 3 kann auch in den Mantel 2 integriert sein. Das Stützskelett 3 kann innen mit gepolstertem textilem Material überzogen sein, damit ein bequemes Tragen der Röntgenschutzbekleidungsanordnung 1 ermöglicht wird.

Die Röntgenschutzbekleidungsanordnung 1 ist an einer zu schützenden Person dargestellt, die einen Korpus aufweist. Der Korpus befindet sich in einem Innenraum des Mantels 2. Der Mantel 2 und das Stützskelett 3 sind dabei so flexibel, dass der Innenraum eine variable Größe hat und sich an unterschiedliche Größen der zu schützenden Person anpassen kann. Der Mantel 2 weist einen unteren Rand 70 auf, aus dem Beine 71 der zu schützenden Person herausragen.

Das Stützskelett 3 ist an seinem in Schwerkraftrichtung unteren Ende mit einer Stützeinrichtung 11 verbunden, die wiederum an ihrem unteren Ende mit einer Rolle 12 versehen ist, die vorzugsweise als Lenkrolle ausgebildet ist. Die Stützeinrichtung 11 ist damit in der Lage, die komplette Gewichtskraft der Röntgenschutzbekleidungsanordnung mit Mantel 2 und Stützskelett 3 in den Fußboden oder eine andere Unterlage, auf der die Beine 71 der zu schützenden Person stehen, abzuleiten.

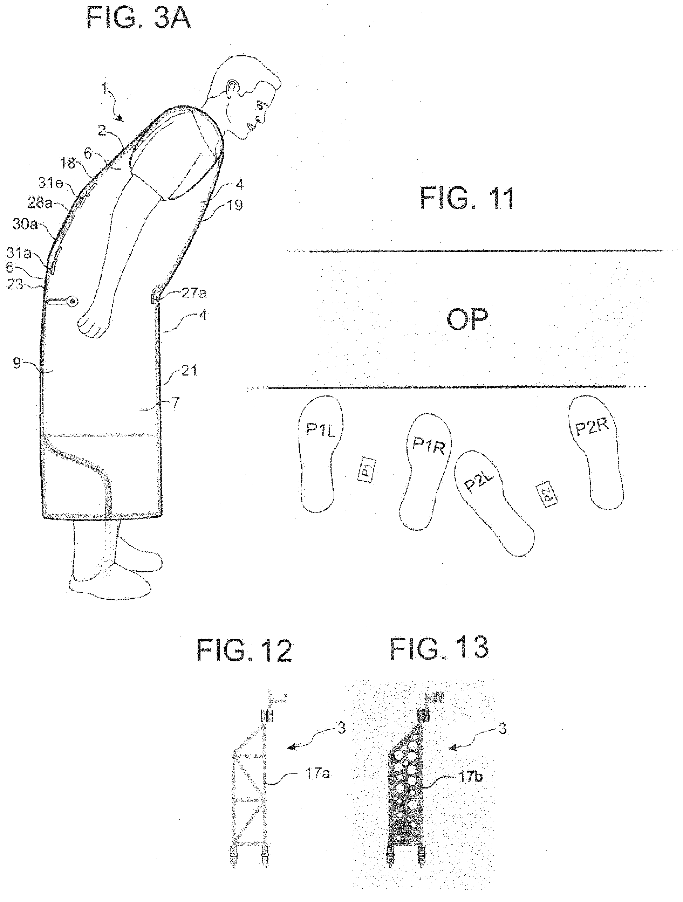

Wie man in Fig. 2 erkennen kann, weist das Stützskelett 3 eine Fachwerkstruktur 16, 17 auf. Fig. 13 zeigt schematisch einen Teil der Fachwerkstruktur 17a. Fig. 14 zeigt alternativ dazu eine Lochblechanordnung 17b, die man anstelle der Fachwerkstruktur 17a verwenden kann.

Das Stützskelett 3 ist mit dem Mantel 2 verbunden und zwar an mehreren Stellen, so dass das Stützskelett 3 das Gewicht des Mantels 2 in Schwerkraftrichtung verteilt an diesen mehreren Stellen aufnehmen kann. Das Stützskelett 3 weist dabei eine in Schwerkraftrichtung nach unten zunehmende lokale Belastbarkeit auf, was weiter unten erläutert werden wird. Damit trägt man der Tatsache Rechnung, dass das Stützskelett in seinem in Schwerkraftrichtung oberen Bereich eine geringere Masse des Mantels 2 tragen muss als in seinem in Schwerkraftrichtung unteren Bereich. Dies bedeutet, dass das Stützskelett 3 in seinem in Schwerkraftrichtung oberen Bereich, also im Bereich der Arme 72 der zu schützenden Person, weniger massiv ausgebildet sein kann, bewegende Arme 72 somit nicht behindert.

Der Mantel 2 weist einen rechten Seitenflügel 4, einen linken Seitenflügel 5 und ein Rückenteil 6 auf. Im betriebsbereiten oder geschlossenen Zustand des Mantels 2 überlappt der linke Seitenflügel 5 den rechten Seitenflügel 4. Zur Fixierung des Mantels 2 im geschlossenen Zustand ist ein oberer Klettverschlussgurt 7 an der Innenseite des linken Seitenflügels 5 befestigt, der zum Verschließen des Mantels 2 auf einem entsprechenden Klettverschlusselement 8 am rechten Seitenflügel 4 befestigt werden kann. Weiter unten kann ein weiterer Klettverschlussgurt 9 am linken Seitenflügel 5 vorgesehen sein, der mit einem entsprechenden Klettverschlusselement 10 auf dem rechten Seitenflügel 4 zusammenwirkt.

Die Klettverschlussgurte 7, 9 können Strahlenschutzmaterial aufweisen.

Das Stützskelett 3 weist eine Schulter-Rücken-Fachwerkstruktur 15 auf, die bogenförmig über den Schultern der zu schützenden Person verläuft, jedoch nicht mit den Schultern in direktem Kontakt steht, wenn die Strahlenschutzbekleidungsanordnung in betriebsbereitem Zustand angelegt worden ist.

Um dies zu realisieren, ist die Stützeinrichtung 11 höhenverstellbar ausgebildet. Damit kann die Röntgenschutzbekleidungsanordnung 1 an die Körpergröße der zu schützenden Person angepasst werden.

Zur Höhenverstellung ist beispielsweise eine Betätigungseinrichtung 13 in Form eines Druckknopfes vorgesehen, die eine Arretierung löst. Um die "korrekte" Höhenlage einzustellen, stellt sich die zu schützende Person auf die Zehenspitzen, um die Röntgenschutzbekleidungsanordnung 1 anzuheben. Die Arretierung wird mit Hilfe der Betätigungseinrichtung 13 gelöst und die Stützeinrichtung 11 bewegt sich durch Schwerkrafteinwirkung nach unten, bis die Stützrolle 12 auf dem Fußboden aufsteht. In diesem Zustand wird die Stützeinrichtung 11 wieder arretiert. Wenn die zu schützende Person dann wieder mit den Fußsohlen auf dem Fußboden aufsteht, ergibt sich ein kleiner Abstand zwischen der Schulter-Rücken-Fachwerkstruktur 15 und der Schulter der zu schützenden Person und es lastet keine Gewichtskraft mehr auf der zu schützenden Person.

Die Betätigungseinrichtung 13 ist beispielsweise durch eine Aussparung 14 in dem unteren Klettverschlusselement 10 geführt und ermöglicht dadurch ebenfalls einen Strahlenschutz im Bereich der Betätigungseinrichtung 13.

Weitere Einzelheiten des Stützskeletts 3 ergeben sich aus Fig. 2.

Wie oben bereits erwähnt, ist der Mantel 2 im vorliegenden Fall an der Außenseite des Stützskeletts 3 befestigt, wobei das Stützskelett 3 die Gewichtskraft des Mantels 2 aufnimmt. Durch die äußere Befestigung des Mantels 2 am Stützskelett 3 ergibt sich eine optische, haptische und funktionelle Einheit.

Es sollte an dieser Stelle angemerkt werden, dass das Stützskelett 3 auch außen am Mantel 2 vorgesehen sein kann. Vorzugsweise ist das Stützskelett 3 allerdings in den Mantel 2 integriert, d.h. es bildet mit dem Mantel 2 eine Einheit.

Das Stützskelett 3 weist eine rechte obere Fachwerkstruktur 16, eine linke obere Fachwerkstruktur 17, die oben erwähnte Schulter-Rücken-Fachwerkstruktur 15, eine rechte untere Fachwerkstruktur 18, eine linke untere Fachwerkstruktur 19 und eine hintere Tragestruktur 23 auf. Die Fachwerkstrukturen 15 bis 19 und 23 sind beispielsweise über Scharniere 26a, b, und 27e-h miteinander verbunden. Anstelle von Scharnieren können auch andere Gelenkverbindungen vorgesehen sein. Die Verbindung zwischen den Fachwerkstrukturen 15 bis 19 und 23 kann auch über verformbare Elemente, beispielsweise Federstäbe oder dergleichen, realisiert werden.

Das Anlegen der Röntgenschutzbekleidungsanordnung 1 kann auf einfache Weise dadurch erfolgen, dass die Seitenflügel 4, 5 aufgeklappt werden. So ermöglichen die Scharniere 26a, 27e, f mittels eines einfachen Handgriffs das Aufklappen des rechten Seitenflügels 4. In entsprechender Weise ermöglichen die Scharniere 26b, 27g, h ein nach außen Klappen des linken Seitenflügels 5. Die Flexibilität der Röntgenschutzbekleidungsanordnung 1 im Rücken- und Hüftbereich wird über den flexiblen Mantel 2 und die flexiblen Eigenschaften des Stützskeletts 3 durch das Zusammenwirken von flexiblen Strukturen in dem Rücken- und Frontbereich vermittelt. Vier Scharniere 27a-d im vorderen Bereich und acht Scharniere 31a-h im Rückenbereich sowie vier Stäbe 28a-d, die gegen vier Schienen 30a-d verschiebbar gelagert sind, ermöglichen eine weitgehende Anpassung der Röntgenschutzbekleidungsanordnung 1 an die Körpergröße einer zu schützenden Person und ermöglichen auch eine entsprechend gute Beweglichkeit der zu schützenden Person innerhalb der Röntgenschutzbekleidungsanordnung.

Wie man in Fig. 3 erkennen kann, ermöglichen die flexiblen Strukturen und die durch das Zusammenwirken der Stäbe 28a-d mit den Schienen 30a-d längenveränderbaren Elemente eine Vorbeugebewegung, d.h. eine Hüftbeugung und eine Beugung in der Lendenwirbelsäule.

Die Stützeinrichtung 11 ist im Bereich einer Mittelebene der Röntgenschutzbekleidungsanordnung 1 angeordnet, also zwischen den Beinen 71 der zu schützenden Person, genauer gesagt außerhalb eines Schwingbereichs der Beine 71. Der Schwingbereich setzt sich aus dem Innenraum über den unteren Rand 70 des Mantels 2 fort. Die Stützeinrichtung 11 behindert dementsprechend nicht eine Bewegung der Beine 71.

Mit anderen Worten ist die Stützeinrichtung innerhalb einer Verlängerung des Innenraums in Schwerkraftrichtung angeordnet.

Damit weist diese Ausgestaltung der Röntgenschutzbekleidungsanordnung 1 einen minimalen Platzbedarf auf und kann auch an relativ engen Orten, wie an einem Operationstisch OP (Fig. 11) getragen werden. Auch ein Stehen auf einem Stufentritt im Operationsraum während einer Operation ist mit diesem kompakten Ausführungsbeispiel möglich. Eine typische Fuß-an-Fuß-Situation, wie in Fig. 11 dargestellt, lässt sich mit der Röntgenschutzbekleidungsanordnung 1 realisieren, so dass mehrere Personen, die jeweils eine Röntgenschutzbekleidungsanordnung 1 tragen, zusammen am Operationstisch OP arbeiten können.

Wie oben beschrieben, ist das Skelett beweglich, d.h. in sich verformbar ausgebildet. Die Beweglichkeit kann dadurch realisiert werden, dass einzelne Elemente gelenkig miteinander verbunden sind oder dadurch, dass Elemente selbst verformbar sind. Wenn man ein in sich verformbares Stützskelett verwendet, dann kann die Stützeinrichtung auch außerhalb der Verlängerung des Innenraums in Schwerkraftrichtung angeordnet sein, wenngleich auch in diesem Fall die Anordnung der Stützeinrichtung innerhalb einer Verlängerung des Innenraums in Schwerkraftrichtung von Vorteil ist.

Fig. 2E zeigt schematisch eine Möglichkeit, wie man die Höhenverstellbarkeit der Stützeinrichtung 11 realisieren kann.

Die Stützeinrichtung 11 ist in der hinteren Fachwerkstruktur 23 verschiebbar gelagert, kann jedoch dort fixiert oder arretiert werden. Dies ist beispielhaft in Fig. 2E dargestellt.

Wenn die zu schützende Person, die die Röntgenschutzbekleidungsanordnung 1 trägt, die Betätigungseinrichtung 13 betätigt, wird ein Haken 36 aus einer Arretierstellung gelöst, so dass die Stützeinrichtung 11 gegenüber der Fachwerkstruktur 23 verschoben werden kann. Nach dem Loslassen der Betätigungseinrichtung 13 wird die Stützeinrichtung 11 wieder gegenüber der Fachwerkstruktur 23 arretiert.

Die Betätigungseinrichtung 13 ist an einem Gehäuse 24 angeordnet, das hier zum Zweck der einfacheren Erläuterung als rundes Gehäuse 24 dargestellt ist. Die Form des Gehäuses 24 spielt in Wirklichkeit eine untergeordnete Rolle. Im runden Gehäuse 24 befindet sich ein Zugdraht 25, dessen eines Ende in dem runden Gehäuse 24 an einer Drahtbefestigung 39 befestigt ist. Das andere Ende des Zugdrahts 29 ist über zwei Gleitringe 38a, b geführt und verlässt dann das Gehäuse 24. Die Betätigungseinrichtung 13 wirkt zwischen den beiden Gleitringen 38a, b auf den Zugdraht. Wenn die Betätigungseinrichtung 13 betätigt wird, wird der Zugdraht 25 in das runde Gehäuse 24 hineingezogen.

Der Zugdraht 25 verläuft über Umlenkeinrichtungen 40a, b in ein anderes Gehäuse 30 im Bereich der Stützeinrichtung 11, das hier als "eckiges" Gehäuse dargestellt ist, ohne dass es auf seine genaue Form ankommt. In dem eckigen Gehäuse ist der Haken 36 unter der Wirkung des Zugdrahts 25 gegen die Wirkung einer Feder 37 verschiebbar. Wenn also die Betätigungseinrichtung 13 betätigt wird, wird der Haken 36 in das eckige Gehäuse 30 hineingezogen. Wenn die Betätigungseinrichtung 13 losgelassen wird, bewegt sich der Haken 36 durch die Kraft der Feder 37 wieder in seine Ausgangsposition zurück.

In Fig. 13 ist erkennbar, dass das Stützskelett 3, von dem ein Teil 17b in Fig. 13 dargestellt ist, eine in Schwerkraftrichtung nach unten zunehmende lokale Belastbarkeit aufweist. Hierzu weist das Teil 17b eine in Schwerkraftrichtung nach unten abnehmende Lochung auf. Die Größe und Anzahl der Löcher nimmt also in Schwerkraftrichtung nach unten hin ab, so dass die gesamte Gewichtskraft der Röntgenschutzbekleidungsanordnung 1 im unteren Bereich der Röntgenschutzbekleidungsanordnung 1 vom Stützskelett 3 aufgenommen wird, um die Stabilitätseigenschaften der Röntgenschutzbekleidungsanordnung zu verbessern.

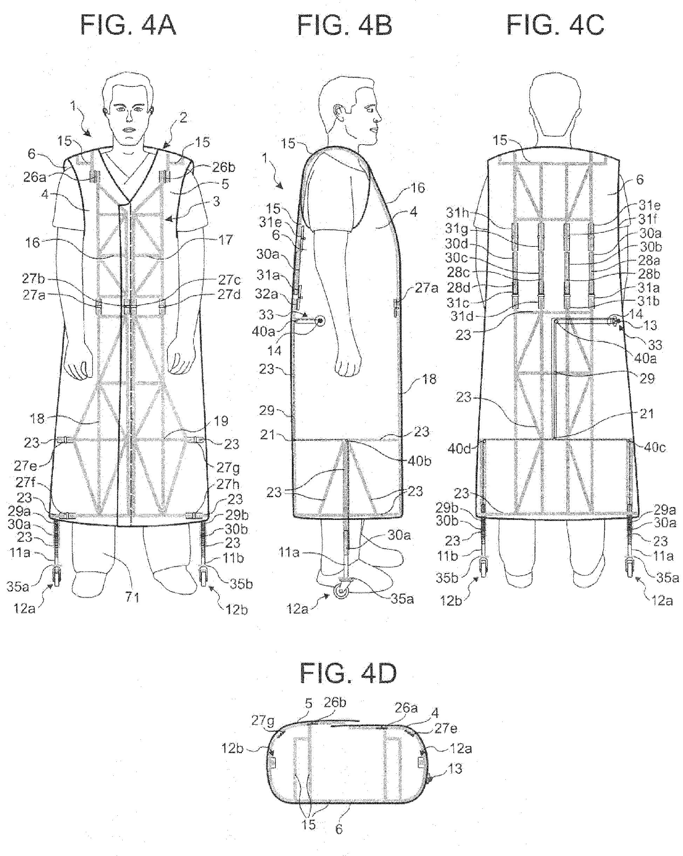

Fig. 4 zeigt eine abgewandelte Ausgestaltung der Röntgenschutzbekleidungsanordnung 1, bei der die Stützeinrichtung 11 auf zwei Stützschienen 11a, 11b aufgeteilt ist, von denen jede an ihrem in Schwerkraftrichtung unteren Ende eine Stützrolle 12a, 12b aufweist. Die Stützrollen 12a, 12b sind vorzugsweise als Lenkrollen ausgebildet und Verbindungselemente 35a, 35b jeweils an den Schienen 11a, 11b befestigt. Die Ausgestaltung ist jedoch so gewählt, dass die Stützeinrichtung 11 mit den Schienen 11a, 11b nicht über dem Mantel 2 in einer Ebene vorsteht, auf der die Schwerkraftrichtung senkrecht steht, mit anderen Worten ist die Stützeinrichtung 11a, 11b innerhalb einer Verlängerung des Innenraums in Schwerkraftrichtung angeordnet. Im vorliegenden Fall ist die Stützeinrichtung mit den Schienen 11a, 11b nicht zwischen den Beinen 71, sondern außerhalb angeordnet. Gleichwohl bleibt ein Schwingbereich für jedes der beiden Beine 71 frei, so dass die Schwingbewegung der Beine 71 im Stehen nach vorne und hinten nach wie vor möglich ist.

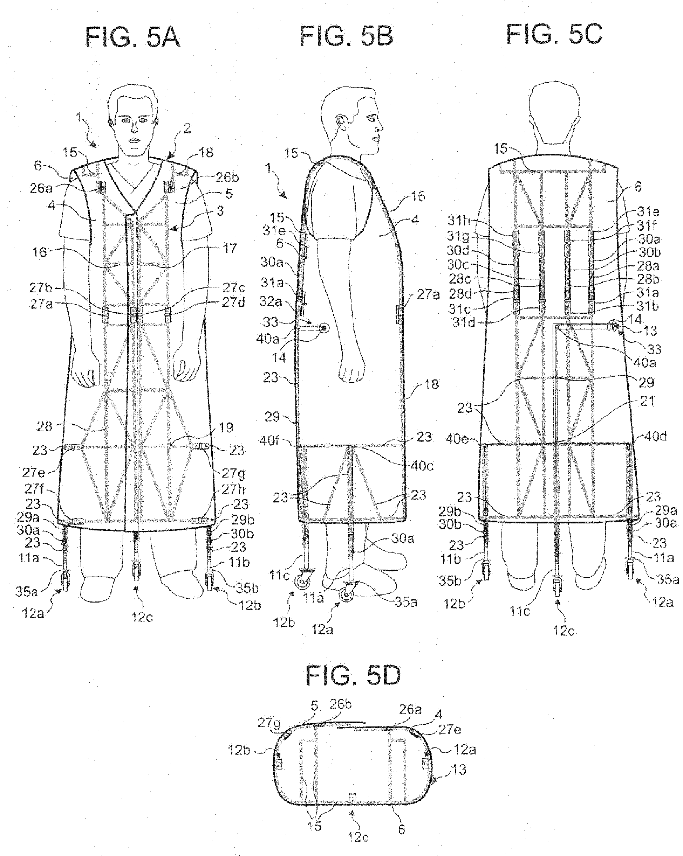

Fig. 5 zeigt eine dritte Ausgestaltung, bei der die Stützeinrichtung 11 drei Schienen 11a, 11b, 11c aufweist, an denen jeweils eine Lenkrolle 12, 12b, 12c über ein Verbindungselement 35a, 35b, 35c befestigt ist. Auch hier ist die Stützeinrichtung mit den Schienen 11a, 11b, 11c vollständig innerhalb einer Verlängerung des Innenraums in Schwerkraftrichtung angeordnet. Die Lenkrollen 12a-12c stehen also in eine Richtung senkrecht zur Schwerkraftrichtung nicht über den Mantel 2 über.

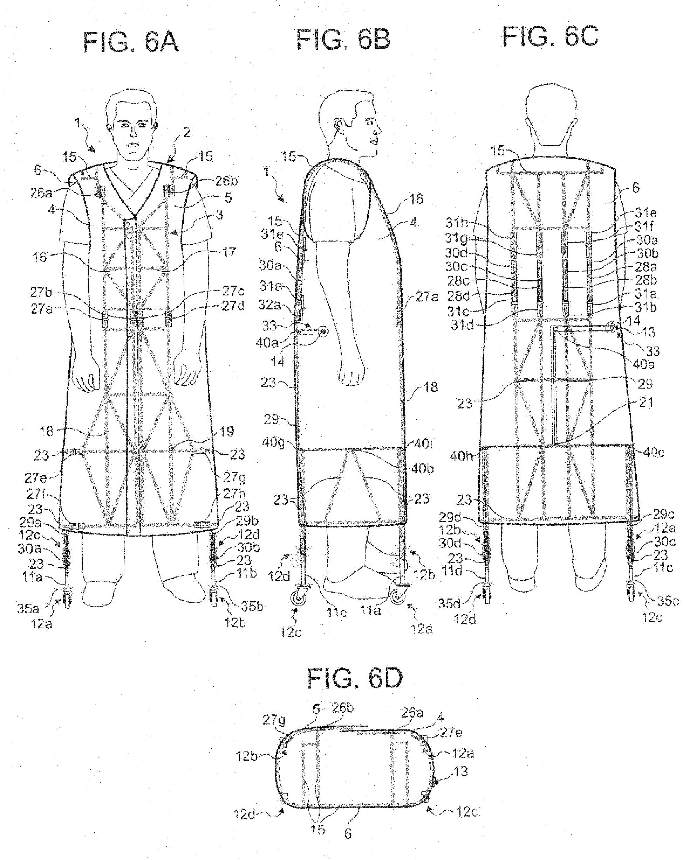

Fig. 6 zeigt eine vierte Ausgestaltung, bei der die Stützeinrichtung 11 nunmehr vier Schienen 11a-11d aufweist, die jeweils über Verbindungselemente 35a-35d mit Lenkrollen 12-12d versehen sind. Auch hier ist sichergestellt, dass die Stützrollen 12a-12d nicht nach außen über den Mantel 2 überstehen, also in eine Ebene senkrecht zur Schwerkraftrichtung.

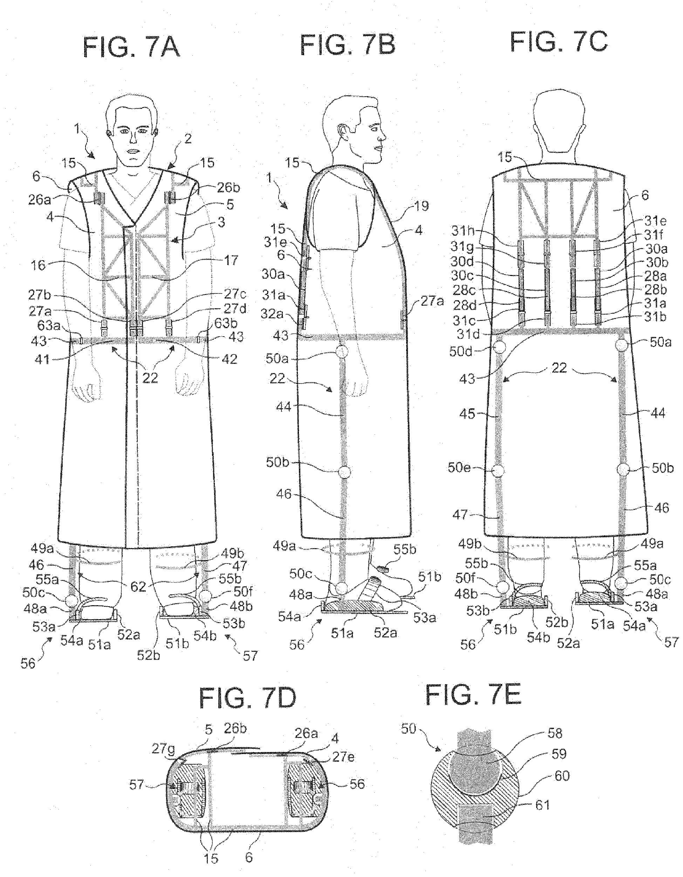

Fig. 7 zeigt schematisch eine fünfte Ausgestaltung der Röntgenschutzbekleidungsanordnung 1, die sich von der Ausgestaltung nach den Fig. 1 bis 6 dadurch unterscheidet, dass die Stützeinrichtung ein Exoskelett 22 für die unteren Extremitäten aufweist. Das Exoskelett 22 weist einen offenen hinteren Hüftring 43 auf, der über zwei Scharniere 63a, 63b am rechten vorderen Hüftring 41 und am linken vorderen Hüftring 42 flexibel befestigt ist. Der hintere Hüftring 43 steht ferner über ein rechtes Kugelgelenk 50a und ein linkes Kugelgelenk 50b in gelenkiger Verbindung mit einer rechten Oberschenkelstange 44 und einer linken Oberschenkelstange 45. Die beiden Oberschenkelstangen 44 und 45 sind wiederum über Kugelgelenke 50b, 50e mit einer rechten Schienenbeinstange 46 und einer linken Schienenbeinstange 47 verbunden. Die Schienenbeinstangen 46, 47 sind über Kugelgelenke 50c, 50f an Fußstangen 48a, 48b befestigt. An den Schienenbeinstangen 46, 47 ist jeweils ein offener Ring 49a, 49b befestigt, über die das Exoskelett 22 am Unterschenkel der zu schützenden Person fixiert ist. Durch Bewegung der Beine der zu schützenden Person, also der Ober- und der Unterschenkel, wird über den offenen Ring 49a, 49b das Exoskelett 22 automatisch mitgeführt. Die Fußstangen 48a, 48b sind jeweils an einem Bodenteil 51a, 51b befestigt. Außerdem können an den Bodenteilen 51, 51b jeweils ein inneres längliches Blech 52a, 52b und ein äußeres längliches Blech 53a, 53b befestigt sein sowie ein hinteres Blech 54a, 54b und ein gebogenes Blech 55a, 55b. Über das Exoskelett 22, das Bestandteil des Stützskeletts 3 ist, wird die Gewichtskraft des Mantels 2 vollständig auf den Boden übertragen. Die zu schützende Person kann ihre Füße durch die offenen Ringe 49a, 49b auf die Bodenteile 51, 51b stellen. Durch die offenen Ringe 49a, 49b und die Bleche 52a, 52b, 53a, 53b, 54a, 54b und 55a, 55b wird das Exoskelett 22 an den unteren Extremitäten der zu schützenden Person fixiert und wird bei der Bewegung der Beine automatisch mitgeführt. Durch die Kugelgelenke 50a, 50f und die Fixierung des Exoskeletts 22 an der unteren Extremität ermöglicht das Exoskelett 22 die freie Beweglichkeit der Hüft-, Knie- und Sprunggelenke, z.B. bei einer Gehbewegung. So kann sich die zu schützende Person mit der Röntgenschutzbekleidungsanordnung 1 physiologisch fortbewegen, ohne dass sie an auf dem Boden stützende, tragende Stützstrukturen stoßen kann oder diese als hinderlich empfinden kann.

Der Begriff "Exoskelett" 22 bedeutet nicht notwendigerweise, dass das Exoskelett tatsächlich außen am Mantel 2 angeordnet ist. Es kann ebenfalls in den Mantel 2 integriert sein.

Fig. 7E zeigt in beispielhafter Ausführungsform ein Kugelgelenk 50, das einen Kugelkopf 58 aufweist, der in eine Kugelpfanne eines Gelenkgehäuses 60 hineinragt. Zwischen dem Kugelkopf 58 und dem Gelenkgehäuse 60 kann eine Lagerschale 59 aus einem elastischen Kunststoff vorgesehen sein. An der dem Kugelkopf 58 gegenüberliegenden Seite ragt ein Stabkopf 61 in einen Hohlraum des Gelenkgehäuses 60 hinein.

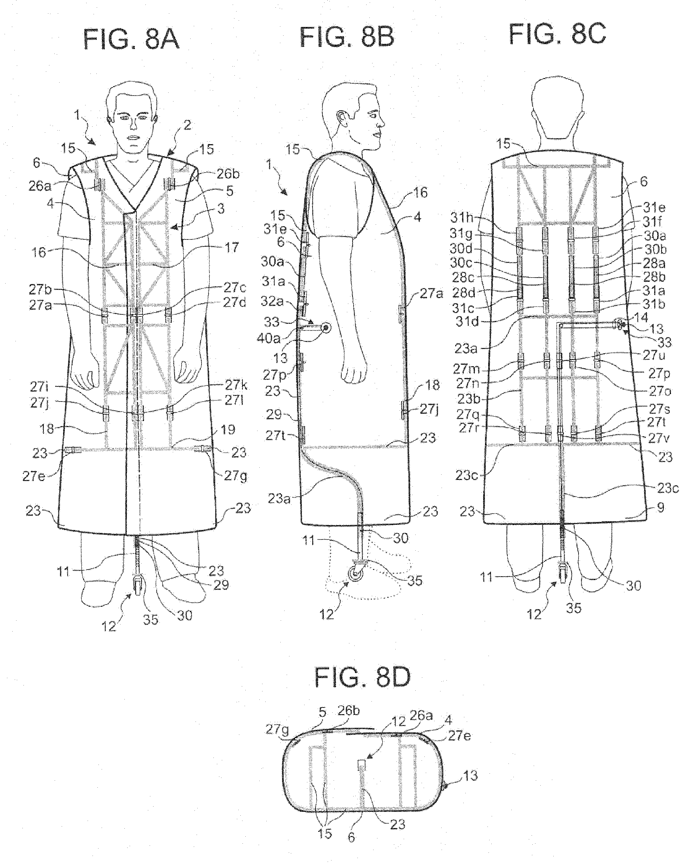

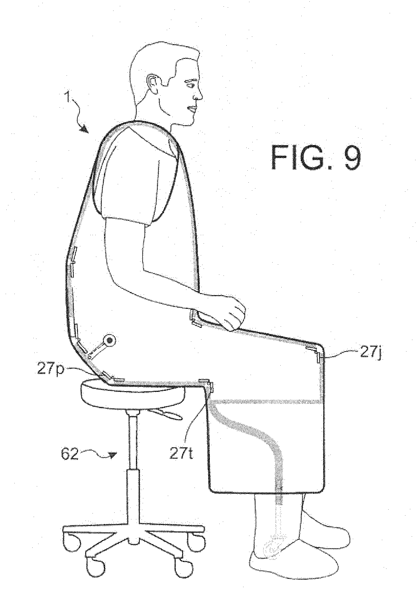

In Fig. 8 ist eine sechste Ausgestaltung der Röntgenschutzbekleidungsanordnung 1 dargestellt, bei der zusätzliche flexible Elemente 27e-27v vorgesehen sind, die für zusätzliche Flexibilität im Hüft- und Kniebereich sorgen, um ein Sitzen zu ermöglichen, beispielsweise auf einem OP-Hocker 62, wie dies in Fig. 9 dargestellt ist.

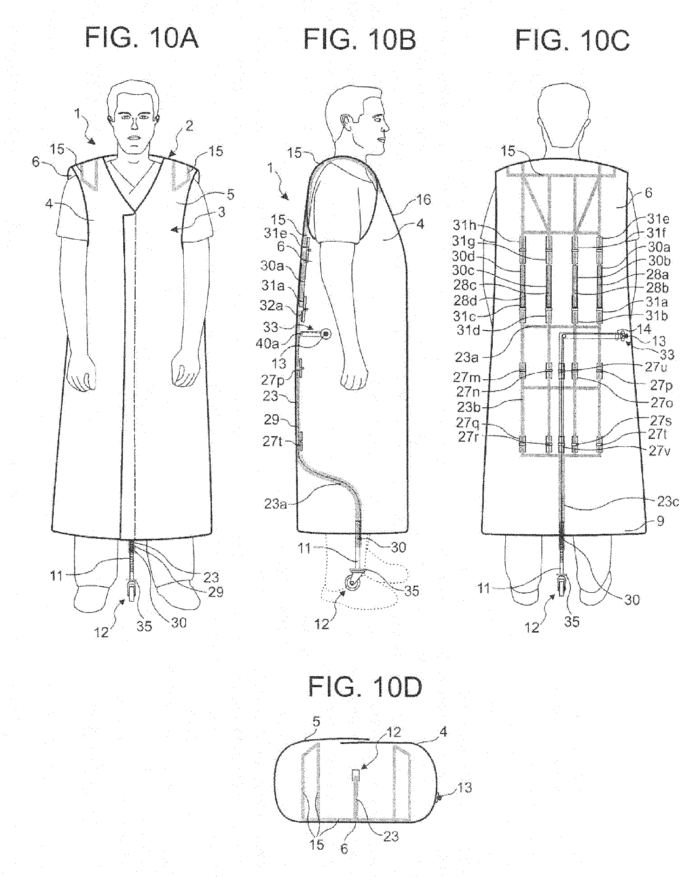

Fig. 10 zeigt eine siebte Ausgestaltung einer Röntgenschutzbekleidungsanordnung 1, bei der aus Vereinfachungsgründen das Stützskelett 3 nur im Rückenbereich vorgesehen ist. Der Komfort und die Knickfestigkeit des Mantels 2 sind im Frontbereich etwas verringert, jedoch werden weniger Teile zur Herstellung benötigt.

In den Fig. 1, 2, 8 bis 10 ist dargestellt, dass die Stützeinrichtung 11 über einen S-förmig geführten Abschnitt 23a der Fachwerkstruktur mit der übrigen Fachwerkstruktur 23 verbunden ist. Damit ist es möglich, die Stützrolle 12 unterhalb eines Massenschwerpunkts der Röntgenschutzbekleidungsanordnung 1 in einer Ebene senkrecht zur Schwerkraftrichtung zu positionieren. Dabei ist von Vorteil, wenn der Mantel 2 und/oder das Stützskelett 3 eine ausgeglichene Massenverteilung um die Stützeinrichtung aufweisen. Im Idealfall ergeben sich dann keine Kippmomente der Röntgenschutzbekleidungsanordnung 1. In der Realität wird allerdings bei der Benutzung der Röntgenschutzbekleidungsanordnung durch die zu schützende Person ein geringes Kippmoment auftreten, das jedoch ohne größere Belastung von der zu schützenden Person aufgenommen werden kann.

Insbesondere im Zusammenhang mit der Beugemöglichkeit, die in Fig. 3 dargestellt ist, kann es von Vorteil sein, wenn zwischen Elementen des Stützskeletts 3 Rückstellmittel vorgesehen sind, beispielsweise eine Feder, die es der zu schützenden Person erleichtern, aus der gebeugten Position wieder in eine aufrechte Position zurückzukehren. Diese Rückstellmittel sind aus Gründen der Übersicht nicht dargestellt.

Im Bereich der längenveränderbaren Elemente 28a-28d, 30a-30d kann der Mantel 2 eine Überlänge aufweisen, die sich dann, wenn sich die zu schützende Person beugt (Fig. 3), glatt zieht.

## Patentansprüche

1. Strahlenschutzbekleidungsanordnung (1) mit einem flexiblen Mantel (2), der Strahlenschutzmaterial aufweist und im Gebrauchszustand einen Innenraum mit einem unteren Rand (70) umgibt, und mit einer Gewichtsentlastungseinrichtung, wobei die Gewichtsentlastungseinrichtung als Stützskelett (3) ausgebildet ist, das mit dem Mantel (2) verbunden ist und das Gewicht des Mantels (2) in Schwerkraftrichtung verteilt an mehreren Stellen aufnimmt, **dadurch gekennzeichnet, dass** der Mantel (2) nicht nur im Schulterbereich an dem Stützsklett (3) aufgehängt ist, sondern mehrere in Schwerkraftrichtung verteilte Befestigungspunkte aufweist, wobei das Stützskelett (3) mit einer im Gebrauchszustand auf einer Unterlage aufstellbaren Stützeinrichtung (11; 46, 47) verbunden ist, die innerhalb einer Verlängerung des Innenraums in Schwerkraftrichtung angeordnet ist und nicht nach außen über den Mantel (2) vorsteht.

2. Strahlenschutzbekleidungsanordnung nach Anspruch 1, **dadurch gekennzeichnet, dass** das Stützskelett (3) beweglich ausgebildet ist.

3. Strahlenschutzbekleidungsanordnung nach Anspruch 1 oder 2, **dadurch gekennzeichnet, dass** die Stützeinrichtung (11) mindestens eine Rolle (12) aufweist.

4. Strahlenschutzbekleidungsanordnung nach einem der Ansprüche 1 bis 3, **dadurch gekennzeichnet, dass** die Stützeinrichtung (44, 45, 46, 47, 48a, b) eine Fußbefestigung (56, 57) und/oder eine Beinbefestigung (49a, b) aufweist.

5. Strahlenschutzbekleidungsanordnung nach einem der Ansprüche 1 bis 4, **dadurch gekennzeichnet, dass** der Mantel (2) und/oder das Stützskelett (3) eine ausgeglichene Massenverteilung um die Stützeinrichtung (11) aufweist.

6. Strahlenschutzbekleidungsanordnung nach einem der Ansprüche 1 bis 5, **dadurch gekennzeichnet, dass** die Stützeinrichtung (11) außerhalb von Beinschwingräumen angeordnet ist, die sich aus dem Innenraum über den unteren Rand (70) hinaus fortsetzen.

7. Strahlenschutzbekleidungsanordnung nach einem der Ansprüche 1 bis 6, **dadurch gekennzeichnet, dass** die Stützeinrichtung (11) höhenverstellbar ist.

8. Strahlenschutzbekleidungsanordnung nach einem der Ansprüche 1 bis 7, **dadurch gekennzeichnet, dass** das Stützskelett (3) zumindest teilweise mit einem textilen Material überzogen ist.

9. Strahlenschutzbekleidungsanordnung nach einem der Ansprüche 1 bis 8, **dadurch gekennzeichnet, dass** das Stützskelett (3) in den Mantel (2) integriert ist.

10. Strahlenschutzbekleidungsanordnung nach einem der Ansprüche 1 bis 9, **dadurch gekennzeichnet, dass** zumindest einige Elemente (28a-d, 30a-d) des Stützskeletts (3) längenveränderbar und/oder verformbar ausgebildet sind.

11. Strahlenschutzbekleidungsanordnung nach Anspruch 10, **dadurch gekennzeichnet, dass** der Mantel (2) im Bereich zumindest eines der längenveränderbaren und/oder verformbaren Elemente (28a-d, 30a-d) eine Überlänge aufweist.

12. Strahlenschutzbekleidungsanordnung nach einem der Ansprüche 1 bis 11, **dadurch gekennzeichnet, dass** das Stützskelett (3) aus Metall und/oder Kunststoff gebildet ist.

13. Strahlenschutzbekleidungsanordnung nach einem der Ansprüche 1 bis 12, **dadurch gekennzeichnet, dass** das Stützskelett (3) eine Fachwerkstruktur (17a) und/oder eine Lochblechanordnung (17b) aufweist.

14. Strahlenschutzbekleidungsanordnung nach einem der Ansprüche 1 bis 13, **dadurch gekennzeichnet, dass** das Stützskelett (3) eine in Schwerkraftrichtung nach unten zunehmende lokale Belastbarkeit aufweist.

15. Strahlenschutzbekleidungsanordnung nach einem der Ansprüche 1 bis 14, **dadurch gekennzeichnet, dass** zumindest eine bewegliche Verbindung zwischen Elementen des Stützskeletts (3) Rückstellmittel aufweist.

## Claims

1. A radiation protection clothing arrangement (1) having a flexible coat (2) which comprises radiation protection material and which, in a state of use, surrounds an interior space with a bottom edge (70), and having a weight-relieving device, **characterized in that** the weight-relieving device is embodied as a support skeleton (3) that is connected to the coat (2) and absorbs the weight of the coat (2) in multiple locations distributed in the direction of gravity, wherein the coat (2) is no longer suspended on the support skeleton (3) solely in the shoulder region, but rather has multiple attachment points that are distributed in the direction of gravity wherein the support skeleton (3) is connected to a support device (11; 46, 47) which can be set up on an underlying surface in a state of use and which is arranged within an extension of the interior space in the direction of gravity and does not protrude outwardly past the coat (2).

2. The radiation protection clothing arrangement according to claim 1, **characterized in that** the support skeleton (3) is embodied to be movable.

3. The radiation protection clothing arrangement according to claim 1 or 2, **characterized in that** the support device (11) comprises at least one caster (12).

4. The radiation protection clothing arrangement according to one of claims 1 to 3, **characterized in that** the support device (44, 45, 46, 47, 48a,b) comprises a foot attachment (56, 57) and/or a leg attachment (49a,b).

5. The radiation protection clothing arrangement according to one of claims 1 to 4, **characterized in that** the coat (2) and/or the support skeleton (3) has a balanced mass distribution around the support device (11).

6. The radiation protection clothing arrangement according to one of claims 1 to 5, **characterized in that** the support device (11) is arranged outside of leg travel spaces that extend out of the interior space past the bottom edge (70).

7. The radiation protection clothing arrangement according to one of claims 1 to 6, **characterized in that** the support device (11) is height-adjustable.

8. The radiation protection clothing arrangement according to one of claims 1 to 7, **characterized in that** the support skeleton (3) is at least partially covered with a textile material.

9. The radiation protection clothing arrangement according to one of claims 1 to 8, **characterized in that** the support skeleton (3) is integrated into the coat (2).

10. The radiation protection clothing arrangement according to one of claims 1 to 9, **characterized in that** at least some elements (28a-d, 30a-d) of the support skeleton (3) are embodied to be length-variable and/or deformable.

11. The radiation protection clothing arrangement according to claim 10, **characterized in that** the coat (2) comprises an excess length in the region of at least one of the length-variable and/or deformable elements (28a-d, 30a-d).

12. The radiation protection clothing arrangement according to one of claims 1 to 11, **characterized in that** the support skeleton (3) is formed from metal and/or plastic.

13. The radiation protection clothing arrangement according to one of claims 1 to 12, **characterized in that** the support skeleton (3) comprises a framework structure (17a) and/or a perforated plate arrangement (17b).

14. The radiation protection clothing arrangement according to one of claims 1 to 13, **characterized in that** the support skeleton (3) has a local load-bearing capacity that increases downwards in the direction of gravity.

15. The radiation protection clothing arrangement according to one of claims 1 to 14, **characterized in that** at least one movable connection between elements of the support skeleton (3) comprises reset means.

## Revendications

1. Système de vêtement de protection contre les rayonnements (1) comprenant un manteau flexible (2) qui présente un matériau de protection contre les rayonnements et dans l'état d'utilisation, entoure un espace intérieur avec un bord inférieur (70), et comprenant un dispositif de soulagement du poids, dans lequel le dispositif de soulagement du poids est conçu en tant que squelette d'appui (3) qui est relié au manteau (2) et reçoit le poids du manteau (2) en plusieurs endroits réparti dans le sens gravitaire, **caractérisé en ce que**
le manteau (2) est suspendu non seulement au niveau des épaules sur le squelette d'appui (3) mais présente plusieurs points de fixation répartis dans le sens gravitaire, dans lequel le squelette d'appui (3) est relié à un dispositif d'appui (11 ; 46, 47) pouvant être placé sur un support dans l'état d'utilisation, qui est disposé à l'intérieur d'un prolongement de l'espace intérieur dans le sens gravitaire et ne fait pas saillie vers l'extérieur sur le manteau (2).

2. Système de vêtement de protection contre les rayonnements selon la revendication 1, **caractérisé en ce que** le squelette d'appui (3) est conçu mobile.

3. Système de vêtement de protection contre les rayonnements selon la revendication 1 ou 2, **caractérisé en ce que** le dispositif d'appui (11) présente au moins un galet (12).

4. Système de vêtement de protection contre les rayonnements selon l'une des revendications 1 à 3, **caractérisé en ce que** le dispositif d'appui (44, 45, 46, 47, 48a, b) présente une fixation pour les pieds (56, 57) et/ou une fixation pour les jambes (49a, b).

5. Système de vêtement de protection contre les rayonnements selon l'une des revendications 1 à 4, **caractérisé en ce que** le manteau (2) et/ou le squelette d'appui (3) présente une répartition de masse équilibrée autour du dispositif d'appui (11).

6. Système de vêtement de protection contre les rayonnements selon l'une des revendications 1 à 5, **caractérisé en ce que** le dispositif d'appui (11) est disposé hors d'espaces de balancement des jambes qui se poursuivent de l'espace intérieur au-delà du bord inférieur (70).

7. Système de vêtement de protection contre les rayonnements selon l'une des revendications 1 à 6, **caractérisé en ce que** le dispositif d'appui (11) est réglable en hauteur.

8. Système de vêtement de protection contre les rayonnements selon l'une des revendications 1 à 7, **caractérisé en ce que** le squelette d'appui (3) est recouvert au moins partiellement d'un matériau textile.

9. Système de vêtement de protection contre les rayonnements selon l'une des revendications 1 à 8, **caractérisé en ce que** le squelette d'appui (3) est intégré dans le manteau (2).

10. Système de vêtement de protection contre les rayonnements selon l'une des revendications 1 à 9, **caractérisé en ce qu'**au moins quelques éléments (28ad, 30a-d) du squelette d'appui (3) sont conçus modifiables en longueur et/ou déformables.

11. Système de vêtement de protection contre les rayonnements selon la revendication 10, **caractérisé en ce que** le manteau (2) présente une surlongueur au niveau d'au moins un des éléments (28a-d, 30a-d) modifiables en longueur et/ou déformables.

12. Système de vêtement de protection contre les rayonnements selon l'une des revendications 1 à 11, **caractérisé en ce que** le squelette d'appui (3) est conçu en métal et/ou en plastique.

13. Système de vêtement de protection contre les rayonnements selon l'une des revendications 1 à 12, **caractérisé en ce que** le squelette d'appui (3) présente une structure à treillis (17a) et/ou un agencement de tôle perforée (17b).

14. Système de vêtement de protection contre les rayonnements selon l'une des revendications 1 à 13, **caractérisé en ce que** le squelette d'appui (3) présente une chargeabilité locale augmentant vers le bas, dans le sens gravitaire.

15. Système de vêtement de protection contre les rayonnements selon l'une des revendications 1 à 14, **caractérisé en ce qu'**au moins une liaison mobile entre des éléments du squelette d'appui (3) présente des moyens de rappel.
